# EUROPEAN PATENT SPECIFICATION

(11) **EP 3 383 284 B1**
(45) Date of publication and mention of the grant of the patent: **28.10.2020**
(21) Application number: 16812788.4
(22) Date of filing: 05.12.2016
(51) Int. Cl.: A61B 17/15, A61B 5/00, A61F 2/46

(54) **ALIGNMENT DEVICE**
AUSRICHTUNGSVORRICHTUNG
DISPOSITIF D'ALIGNEMENT

(30) Priority: 03.12.2015 US 201562262695 P
(43) Date of publication of application: 10.10.2018
(73) Proprietor: Agarwal, Sanjeev, Lisvane Cardiff CF14 0RS (GB); Agarwal, Rajiv, Lucknow 220 020 (IN)
(72) Inventor: Agarwal, Sanjeev, Lisvane Cardiff CF14 0RS (GB); Agarwal, Rajiv, Lucknow 220 020 (IN)
(74) Representative: Murgitroyd & Company
(86) International application number: PCT/GB2016/053832
(87) International publication number: WO 2017/093769

(56) References cited:
- WO-A1-2013/173700
- WO-A1-2015/038979
- US-A1- 2003 212 403
- US-A1- 2005 113 846
- US-A1- 2011 213 221

## Description

### FIELD OF THE INVENTION

The present invention relates generally to the field of orthopaedics, specifically, knee replacement surgery and alignment devices for knee replacement surgery.

### BACKGROUND

The knee is a large and complex joint in the human body which permits flexion, extension, rotation, as well as sliding between the femur and tibia. The knee can be subject to forces many times the weight of the body, and is therefore vulnerable to both acute injury and the development of degenerative conditions such as osteoarthritis.

Knee replacement, or knee arthroplasty, is a surgical procedure to replace the weight-bearing surfaces of the knee joint. Knee replacement surgery is performed in order to relieve pain and disability associated with conditions such as those mentioned above.

Knee replacement surgery can be performed as a partial or a total knee replacement. Typically, the surgery involves the replacement of unhealthy or damaged joint surfaces of the knee with metal and plastic components, which provide pain relief and are designed to allow continued motion of the knee after surgery.

Due to its complex nature and the interplay between the various elements of the knee joint, ensuring that any components used in a partial or total replacement knee are properly aligned is critical. Part of this includes ensuring that the femur and tibia are aligned correctly when the replacement components are *in situ* since a small variation from the mechanical or anatomical axes of the leg can result in early failure of the prosthesis.

Document US2011/213221 A1 discloses an exemplary alignment device for aligning femoral and tibial bones at a knee joint.

### SUMMARY

The present disclosure relates to an alignment device as defined in claim 1.

The disclosed alignment device is designed for aligning the femur and tibia at the knee joint, the device includes a femoral part, a tibial part, and optionally a freehand sensor. The femoral part may have two components. The two components may be an intramedullary sensor and an extramedullary sensor. The femoral sensor can either be inserted into the canal or fixed on the exposed femur at the time of knee replacement. The intramedullary component is used where the femoral canal is available and accessible to the surgeon.

The extramedullary sensor is used where the femoral canal is not available for instrumentation due to presence of metalwork or deformity.

The tibial part is mounted on the leg parallel to the tibia. The tibial part will communicate with the femoral sensor, and help the operator to ensure accurate alignment of the tibia and femur. The relative alignment is displayed on the screen attached to the tibial component, or on a remote computer.

The freehand sensor is also connected wirelessly to the tibial and femoral components. This can be used to check the alignment of the trial in relation to the femoral axis and the tibial axis. This can provide both varus - valgus, sagittal and rotational alignment information.

There is disclosed herein a method for measuring alignment characteristics of a prosthetic knee joint arranged between a femur and tibia. The method may include obtaining an alignment device as claimed and coupling the alignment device to the femur and tibia and determining a first range of motion. The method may further include performing a knee replacement operation and determining a second range of motion

These features and advantages of this invention will become apparent from the following detailed description taken in conjunction with the accompanying drawings.

### BRIEF DESCRIPTION OF THE DRAWINGS

The accompanying drawings, which are incorporated in and constitute a part of the specification, illustrate embodiments of the invention and together with the detailed description herein, serve to explain the principles of the invention. It is emphasized that, in accordance with the standard practice in the industry, various features are not drawn to scale. In fact, the dimensions of the various features may be arbitrarily increased or reduced for clarity of discussion. The drawings are only for purposes of illustrating preferred embodiments and are not to be construed as limiting the invention.

An embodiment will now be described, by way of example only, and with reference to the accompanying drawings, in which:
FIG. 1 is a side view of the alignment device, using an intramedullary sensor and positioned on a portion of a patient's leg, in accordance with an aspect of the present invention;
FIG. 2 is a frontal view of the alignment device of FIG. 1, in accordance with an aspect of the present invention;
FIG. 3 is a side view of the alignment device, using an extramedullary sensor and positioned on a portion of a patient's leg, in accordance with an aspect of the present invention;
FIG. 4 is a frontal view of the alignment device of FIG. 3, in accordance with an aspect of the present invention;
FIG. 5 shows the freehand sensor and its application to the distal end of trial femoral component, in the frontal view, in accordance with an aspect of the present invention;
FIG. 6 shows a side view of the freehand sensor and its application to the femoral trial component, in accordance with an aspect of the present invention;
FIG. 7 shows the application of the freehand sensor to the tibial cut surface and, on the surface of the trial tibial component, in accordance with an aspect of the present invention; and
FIG. 8 depicts one example of a method for using an alignment device according to the present invention.

### DETAILED DESCRIPTION

Generally stated, disclosed herein is an embodiment of an alignment guide. Further, a method (not claimed) for using the alignment guide is discussed.

In this detailed description and the following claims, the words proximal, distal, anterior, posterior, medial, lateral, superior, inferior, dorsal and plantar are defined by their standard usage for indicating a particular part of a bone or implant according to the relative disposition of the natural bone or directional terms of reference. For example, "proximal" means the portion of the guide or bone nearest the torso, while "distal" indicates the portion of the guide or bone farthest from the torso. As for directional terms, "anterior" is a direction towards the front side of the body, "posterior" means a direction towards the back side of the body, "medial" means towards the midline of the body, "lateral" is a direction towards the sides or away from the midline of the body, "superior" means a direction above and "inferior" means a direction below another object or structure. Further, specifically in regards to the foot, the term "dorsal" refers to the top of the foot and the term "plantar" refers to the bottom of the foot.

The alignment of the knee with respect to the hip and the ankle is determined by measuring the angle between the mechanical axis of the femur and the mechanical axis of the tibia. The mechanical axis of femur makes an angle of 5 to 7 degrees with the anatomical axis of the femur. The mechanical axis of tibia is considered to be collinear with the anatomical axis. When the natural alignment and orientation of the knee joint is disrupted, subsequent joint instability, imbalance and stress at fixation interfaces can occur. Correction of this type of damage can require surgical replacement of some, or all, of the affected knee joint.

Ideally, the knee replacement operation should restore the mechanical axis of the lower limb, such that the line drawn from the centre of the hip to the centre of the ankle should pass through the centre of the knee. While this is the goal of knee replacement surgery, it is important to note that there are wide variations in the mechanical axis of different individuals.

Typically, knee replacement surgery involves exposure of the front of the knee allowing exposure of the distal (lower) end of the femur and the proximal (upper) end of the tibia. The ends of these bones are then accurately cut to shape using cutting guides oriented to the long axis of the bones. Metal components are then impacted onto the bone using a suitable cement or cementless technique.

Conventional knee replacement instruments allow accurate measurement of positioning of the cutting jigs in relation to the anatomical axis of the femur and tibia. However, they do not allow checking of alignment once bone cuts have been made. In addition, alignment of the knee in side profile should demonstrate that the knee is able to fully straighten (extend) after an operation. Conventional mechanical instruments do not have a system to measure this, or to measure the range of flexion achieved.

It is desirable that rotation of the femoral component and the tibial component should be closely matched. Errors of tibial rotation can lead to early failure and a high revision rate of a prosthesis.

Alignment is thus an important surgeon-controlled factor in determining the durability of a knee replacement since any significant variation from normal alignment will usually result in pain and discomfort for the patient as well as a tendency for components of the joint replacement to wear more quickly than otherwise.

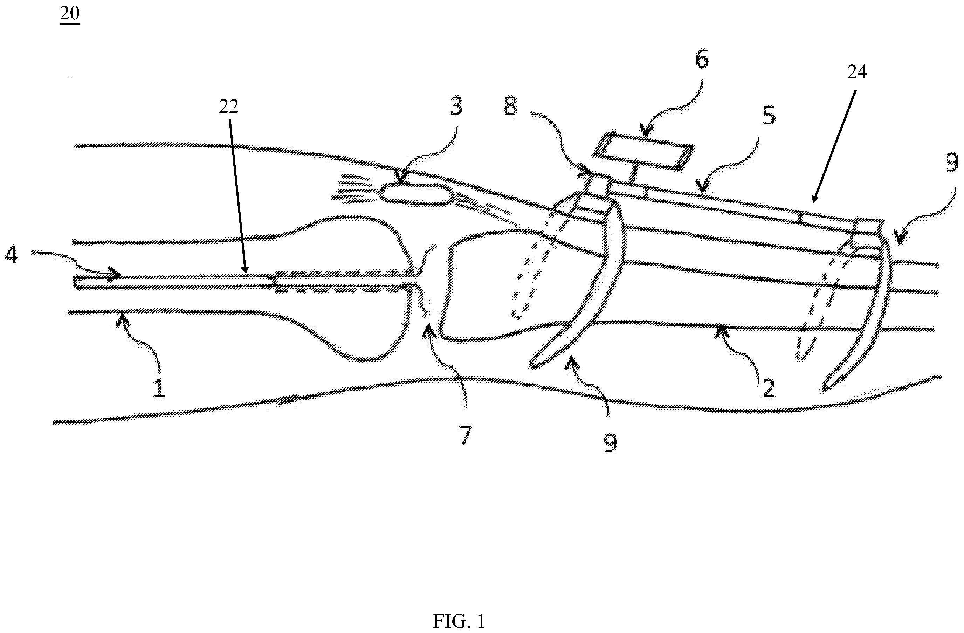

In order to achieve acceptable alignment, the accurate placement of the components and the relative positioning of the knee joint with respect to the hips and ankle is key. According to an example and as shown in FIGS. 1-7, there is provided alignment devices **20**, **30** for aligning a femoral bone **1** and a tibial bone **2** at a knee joint **3.** The alignment devices 20, 30 permit various translational, angulation and rotational aspects to be measured. If desired alignment has not been achieved, further bone and soft tissue procedures can be undertaken to achieve the correct alignment. The devices 20, 30 will allow rechecking of the alignment and the accuracy of the bone cuts. It will also allow for checking of the alignment once the trial components, or the actual implant is in position.

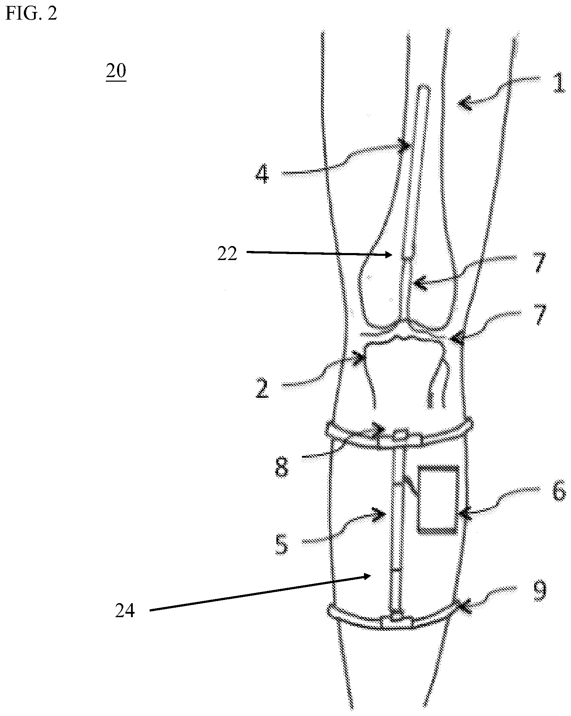

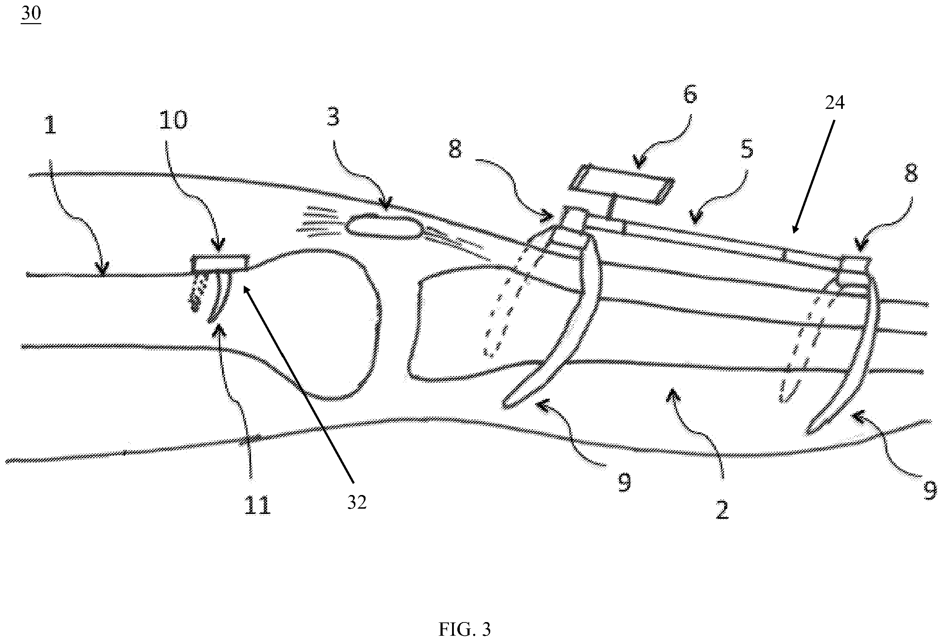

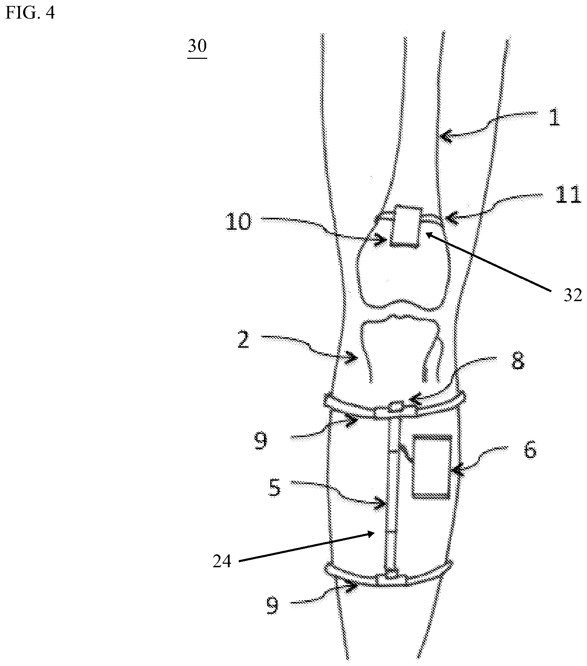

The devices 20, 30 include a femoral portion **22**, **32**, a tibial portion **24**, and a freehand sensor **12.** The femoral portion 22, 32 can be of two types, and each can be used individually or both femoral parts can be used in conjunction with each other. The two types are a sensor rod **4**, as described in greater detail below with reference to FIGS. 1 and 2, and a flat sensor device **10**, as described in greater detail below with reference to FIGS. 3 and 4.

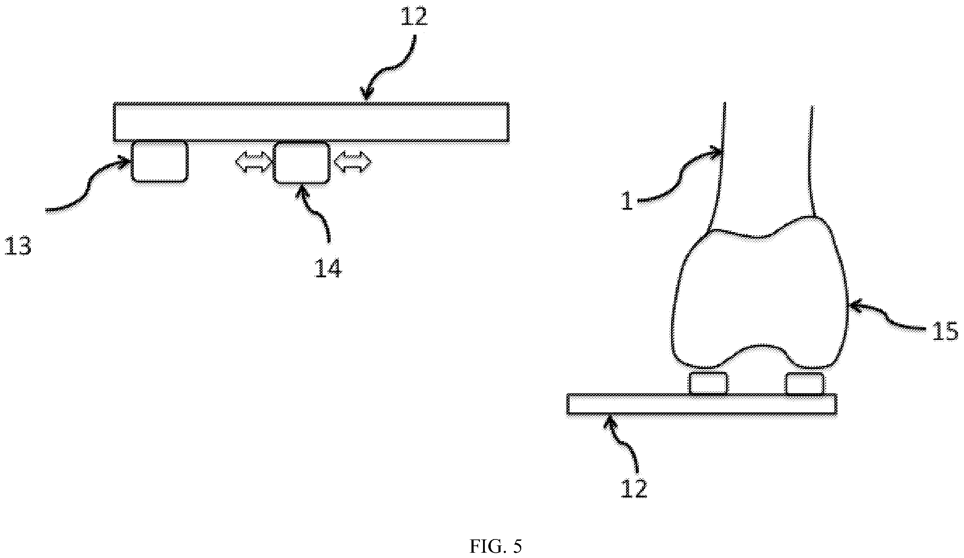

FIGS. 1-7 show portions of example alignment devices 20, 30 according to one embodiment. As shown in FIGS. 1 and 2, the device 20 includes a femoral portion 22, a tibial portion 24, and a freehand sensor 12, as shown in FIGS. 5-7. The femoral portion 22 of the device 20 includes a sensor rod 4. The sensor rod 4 may be, for example, a rod which acts as a housing for a sensor (not shown). The sensor (not shown) may be positioned inside the rod 4. The sensor (not shown) may also be collinear with the rod 4, such that the position of the rod 4 matches the alignment of the sensor. The rod 4 can be inserted retrograde (from the lower end of femur) into the femoral intramedullary canal. The rod 4 has a tape or chord **7** at one end which can be used to aid its removal from the canal. The rod 4 is henceforth called femoral intramedullary sensor ("FIS") 4. Once inserted into the femur 1, the FIS 4 acts as a reference for the anatomical axis of the femoral shaft.

As shown in FIGS. 3 and 4, the device 30 includes a femoral portion 32 and a tibial portion 24. The femoral portion 32 of the device 30 includes a flat sensor device 10. The flat sensor device 10 may include, for example, a housing and a sensor (not shown) positioned within the housing. The sensor 10 within the housing will have at least one of a three-axis accelerometer and a three-axis gyroscope. At least one of the sensors 4, 10 may be configured to monitor the orientation in the three-dimensional coordinate reference system and to generate orientation data corresponding to the orientation of sensor 4 or sensor 10. The flat sensor device 10 may attach on the front of the patient's femur to position the sensor (not shown) for extramedullary alignment.

The flat device 10 can be fixed to the surface of the femur 1. Typically, this would be the anterior (front) aspect, but it can be on any side or part of the femur 1. The flat device 10 has a fixation mechanism **11** to fix the flat device 10 to the femur 1. The clamp 11 on the housing of the extramedullary sensor 10 may be, for example, designed to include screws, clamps, pegs or any other suitable method of fixation. This is henceforth referred to as femoral extramedullary sensor ("FES") 10.

Each of the sensor rod 4 and sensor device 10 may have at least one of a three-axis accelerometer and a three-axis gyroscope. At least one of the sensor rod 4 and sensor device 10 may be configured to monitor the orientation in a three dimensional coordinate reference system and to generate orientation data corresponding to the orientation of the rod 4 and/or device 10. The rod 4 may be a housing to hold a sensor. The sensor may be collinear with the rod 4, thereby, allowing for the position of the rod 4 to match the alignment of the sensor. Similarly for the extramedullary sensor 10, the attachment on the front of the femur 1 is the housing for the sensor 4, 10 for extramedullary alignment.

Although not shown, it is also contemplated that the alignment devices 20, 30 may include both a sensor rod 4, as described in greater detail above with reference to FIGS. 1 and 2, and a flat sensor device 10, as described in greater detail above with reference to FIGS. 3 and 4.

The tibial portion 24 of the devices 20, 30 include a sensor rod **5** mounted on two clamps **8.** The sensor rod 5 may include, for example, a housing and a sensor (not shown). The sensor (not shown) may be included in the housing of the sensor rod 5. The axis of the sensor (not shown) and the housing may be positioned collinear to allow the axis of the sensor (not shown) to be matched to the anatomic axis of the tibia when the rod 5 is aligned to the anatomic tibial axis. The sensor rod 5 within the housing may have, for example, at least one of a three-axis accelerometer and a three-axis gyroscope. The sensor rod 5 may be, for example, configured to monitor orientation in the three dimensional coordinate reference system and to generate orientation data corresponding to the orientation of the sensor rod 5. The axis of the sensor 5 and the housing may be, for example, collinear. In one embodiment, the axis of the sensor 5 can be matched to the tibial axis if the rod 5 is matched to the tibial axis. The housing of the sensor rod 5 may be attached to the patient's leg by, for example, clamps **8** including pegs, screws or other fasteners.

The clamps 8 allow adjustment of the position of the sensor rod 5, so that it can be aligned in relation to the tibia 2, or the mechanical axis of the patient's leg. The clamps 8 may include, for example, spring loaded arms that may be opened and closed around the patient's leg. It is also contemplated that the clamps 8 may be, for example, two separate arms that may be attached together around the patient's leg to position and secure the sensor rod 5 to the patient. Further, it is also contemplated that the clamps may be, for example, an elastic band or strap that extends around the patient's leg and fastened together at each end by, for example, a hook, clasp, or other fastener, or any other method for attachment to allow for stable contact.

The clamps 8 also may include a movable joint positioned between the clamps 8 and the housing of the sensor rod 5. The movable joint allows the sensor rod 5 to be pulled forward or backwards, on either side (medially and laterally) or proximally / distally (up or down) while the clamps 8 remain fixed to the patient's leg.

The clamps 8 are attached to two curved arms **9**, which help to secure the tibial portion 24 to the leg. It is intended that, once the curved arms 9 and clamps 8 are mounted, the tibial sensor 5 is parallel to the shaft of the tibia 2.

The sensor rod 5 is connected to a screen **6** by any known means. The screen 6 displays the relationship of the femoral sensors 4 and/or 10 to the tibial sensor 5. The screen 6 may display, for example, an image showing the position of the sensors 4 and/or 10 and the patient's leg, as well as numbers indicating the angle, in degrees, between the two sensors.

The connection can be articulated to allow convenient positioning of the sensor rod 5, so that it can be visualised by the operator. It may be equipped with auditory signals which indicate when the femoral sensors 4 and/or 10 are aligned with the tibial sensor 5. The auditory signals would be broadcast through a speaker (not shown) that may be contained within the housing of the display 6.

It would be possible to connect the display 6 to an external computer for the purpose of storage, processing, display or printing of information. The display unit 6 may be directly incorporated into the sensor 5 so as to make it a combined unit. The sensor rod 4, flat sensor device 10, and the sensor rod 5 may each communicate wirelessly, be wired, or a combination of wired and wirelessly with the display 6.

In FIGS. 1 and 2, the femoral intramedullary sensor 4 has a tape 7 fixed at one end. This can be either a single strand or more than one strand and the tape 7 may be useful in extraction of the rod 4 before final implantation of the femoral component.

The shape of the femoral intramedullary sensor 4 is intended to be, for example, cylindrical, although any other shape which can be accommodated in the femoral canal may also be used. The sensor 4 may be expansible to contour itself to the shape of the medullary canal. The entire alignment instrument 20, 30 can be made of material which can be sterilised for use during surgery. In addition, portions of the alignment instruments 20, 30 may be made of a sterilisable or disposable material. The femoral intramedullary sensor 4 can be removed and reinserted into the medullary canal multiple times during a knee replacement procedure in order to permit adjustments to bone cuts or prostheses positioning. Insertion and removal can be effected whilst the knee joint under consideration is bent at an angle, such as 90° for example, thereby exposing the canal for insertion/removal.

A channel is made into the femur as part of the standard technique in knee replacement and the same channel can be used for the femoral intramedullary sensor 4.

The alignment devices 20, 30 may also include a freehand sensor 12, as shown in FIGS. 5-7. The freehand sensor 12 may include a housing and a sensor. The sensor 12 may have at least one of a three-axis accelerometer and a three axis gyroscope. The sensor 12 may be, for example, configured to monitor orientation in the three dimensional coordinate reference system and to generate orientation data corresponding to the orientation of the rod 12.

As shown in FIG. 5, the freehand sensor 12 includes two blocks **13**, **14** which are used to place it against the surface whose alignment is being measured. Block 13 may have a fixed attachment to the rod 12. The freehand sensor 12 includes a rod for housing for the sensor (not shown). The blocks 13, 14 allow for placement of the freehand sensor rod 12 along the axis of the blocks 13, 14. Each block 13, 14 may be held apposed to the femoral or tibial trial, for example, one block 13, 14 may contact the medial side and the other block 13, 14 may contact the lateral side. In one embodiment, block 13 may be fixed and block 14 may be mobile as femoral and tibial trial components of different sizes are tried in the patient's bones. Block 14 may be mobile side to side to allow placement against the femoral and tibial trial components of different sizes. Block 14 may move within, for example, a track in the rod 12 which is aligned for side to side motion of the block 14. The blocks 13, 14 may contact, for example, the distal most point on the femoral trial on the medial and lateral sides to allow for a determination of the varus or valgus angle of the femoral trial prosthesis in relation to the intramedullary rod sensor 4. The blocks 13, 14 may also be apposed to the most anterior point on the femoral condyles of the trial. The positioning of the blocks 13, 14 allow for the rotation of the femoral component to be compared to the tibial rotation. Further, on the tibial side, the blocks 13, 14 can be placed such that one is in contact with the cut surface of the tibia on the medial side and the other is on the lateral side to enable the varus and valgus alignment of the tibial trial prosthesis to be obtained. If the rod 12 is placed in an anterior-posterior direction on the cut surface of tibia, the posterior slope on the tibia can be determined in relation to the tibial axis given by rod 12.

The freehand sensor 12 can be placed against the distal (lower) end of the femoral component and this will allow measurement of the varus and valgus angle of femoral trial component **15** in relation to the intramedullary sensor 4.

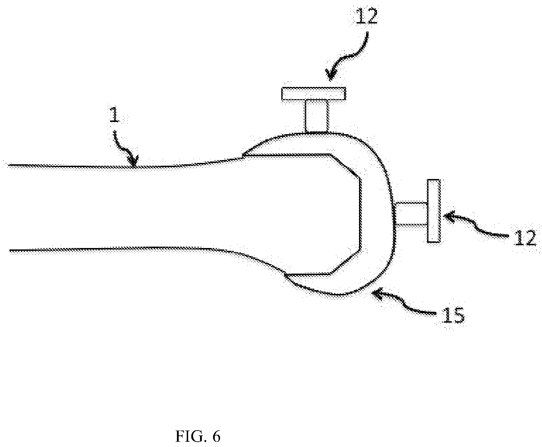

Referring now to FIG. 6, the sensor 12 can also be placed on the anterior surface of femoral trial component 15 to show the rotation of the femoral trial 15. The sensor 12 may be held against the femoral trial component 15 while measurements are taken.

The tibial trial can be matched to femoral trial 15 on the basis of this measurement.

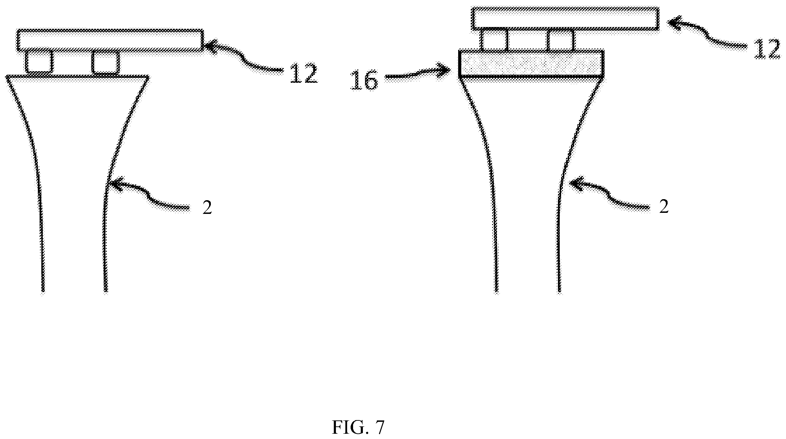

As shown in FIG. 7, the freehand sensor 12 may also be placed on the cut surface of tibia 1, and this allows checking the varus or valgus alignment of the tibial cut in relation to the tibial axis. Additionally, the tibial trial **16** can be placed on the tibial surface and alignment checked.

The device, with its various components, allows checking of a range of alignment indices in knee replacement surgery. If the desired alignment is not achieved, further surgical procedures using traditional instruments are carried out to correct the alignment and the devices 20, 30 reused to demonstrate the final result.

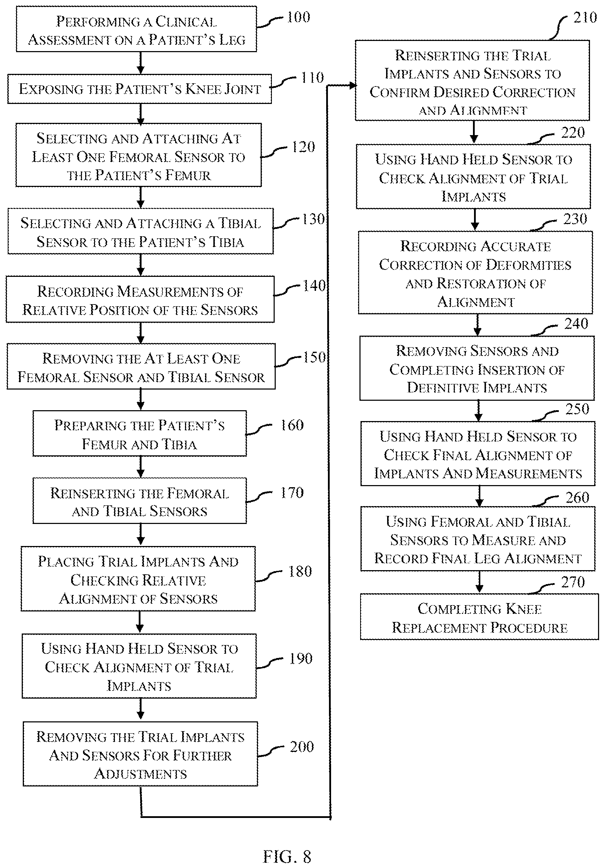

A method of using an alignment device 20, 30 is shown in FIG. 8 and described in greater detail below. The method may include, for example, performing a clinical assessment on a patient's leg **100**, surgical exposure of the knee joint **110**, selecting and attaching at least one femoral sensor to the patient's femur **120**, selecting and attaching to tibial sensor to the patient's tibia **130**, recording measurements of the relative position of the sensors **140**, removing at least one femoral sensor and tibial sensor **150**, preparing the patient's femur and tibia **160**, reinserting the femoral and tibial sensors **170**, placement of trial implants and checking the relative alignment of sensors **180**, using the handheld sensor to check the alignment of trial implants **190**, removal of trial implants and sensors for making any further adjustments using standard instruments to achieve the desired collection of deformities **200**, reinsertion of sensors and trial implants to ensure desired correction and alignment has been achieved **210**, using the handheld sensor to again check alignment of trial implants **220**, recording accurate correction of deformities and restoration of alignment **230**, removing the femoral and tibial sensors to complete insertion of definitive implant **240**, using the hand held sensor to check the final alignment of implants and record the measurements **250**, and using the femoral and tibial sensors to measure and record the final leg alignment **260.**

With continued reference to FIG. 8, a clinical assessment is made to check for limb alignment, any fixed flexion deformities, range of flexion and various other parameters prior to the knee replacement operation.

At the time of the knee replacement operation, the initial step is exposure of the knee. The preparation of the femur 1 can be done in various ways. Traditional femoral jigs involve making a hole in the distal femur to gain entry into the medullary canal. In this situation, the femoral intramedullary sensor 4 may be employed. With the development of patient specific instruments, a femoral canal entry is not required, and in that situation the extramedullary sensor 10 may be used. In addition, with patients that have an obstructed or occupied femoral canal from, for example, previous trauma or surgery, an extramedullary sensor 10 may be used. Such trauma or surgery situations may include, but are not limited to, femoral intramedullary rods, previous trauma, femoral stems from an existing knee replacement, long stem femoral components from the hip, or metalwork used in fracture stabilization.

If the femoral sensor 4 is selected, then the appropriate sized femoral sensor 4 is inserted into the patient. Next, the tibial sensor 5 is mounted on the leg and the relative position of the two sensors 4 and 5 is checked. This should be done in full extension (with the leg as straight as possible) and in flexion (with the knee bent fully). As the relative position of sensors 4, 5 is checked, information such as, (1) range of movement of the knee (flexion and extension range); and (2) varus and valgus stability on stressing the knee in full extension and in different degrees of flexion, may be recorded. The relative position information or data may be recorded either electronically or printed in hard copy.

If the femoral extramedullary sensor 10 is selected, then the femoral extramedullary sensor 10 can be calibrated with regards to the intramedullary sensor 4. Once the extramedullary sensor 10 is calibrated, the intramedullary sensor 4 may be removed. The extramedullary sensor 10 may be used along with the tibial sensor 5, without the need for intramedullary sensor 4. This would be useful in situations where the femoral canal is obstructed or occupied.

When the femoral canal is obstructed or occupied, the extramedullary sensor 10 may be used as a reference, without calibration by placing the sensor 10 along the anterior surface of femur. Alternatively, the sensor 10 may be used in computer navigation surgery to find the centre of the femoral head. The centre of the femoral head may be found by placing the sensor 10 on the patient's femur and moving the femur in different planes. The data obtained from the sensor 10 while the patient's femur is moved may then be used to deduce the centre of the femoral head.

An example of when the femoral canal may be obstructed is revision knee surgery using femoral stems. The relative position of the extramedullary sensor 10 and the tibial sensor 5 may be checked. The relative positions may be checked by moving the leg from full extension to flexion. As the leg is moved, the relative position of sensors 10, 5 may be used to obtain the range of movement of the knee and the varus/valgus stability on stressing the knee.

The clamp 11 on the femoral extramedullary sensor 10 allows adjustment in position such that the extramedullary sensor 10 can be aligned with the intramedullary sensor 4 when both sensors 4, 10 are used. Each sensor 4, 10 can be detected individually, or in combination, by the tibial sensor 5.

After this, the femoral sensor(s) 4, 10 and tibial sensor 5 are removed and the knee replacement operation proceeds with standard instruments. Appropriate bone cuts are made to the femur 1 and tibia 2. Once the cuts are made in the bones 1, 2, trial implants 15, 16, as shown in FIGS. 6-7, may be placed to check balancing clinically. At this stage, the femoral sensor(s) 4, 10 and tibial sensor 5 are again mounted with the trial implants 15, 16 in situ, another check is made for the parameters which were recorded initially. This includes range of movement and stability of the knee in varus and valgus stressing. The information gained from the device helps confirm clinical assessment. This information from the device is also recorded. If there is residual flexion deformity, or ligament imbalance in the knee, these can be corrected through the standard surgical techniques. The trials 15, 16 are mounted with the device 20, 30 and alignment is rechecked using the femoral and tibial sensors 4 and/or 10, 5.

The freehand sensor 12 may be used to check the alignment of the femoral and tibial trial components 15, 16, as well as the actual femoral and tibial joint replacement components. The freehand sensor 12 may be used after the implant components have been inserted into the patient's leg to determine whether the final alignment of the components matches the desired alignment. For example, the sensor 12 may be placed at the distal end of the femoral component to determine the varus or valgus alignment of the femoral component. Similarly, the sensor 12 may be placed on the tibial component to determine the varus or valgus alignment and to measure the posterior slope of the tibia.

This application comprises the principle of detecting the position of one sensor in relation to another. In alternative embodiments, the devices disclosed herein can be applied to any areas of surgery whereby the surgeon wishes to align, or determine the relative alignment of two instruments or bones, or a combination thereof.

For instance, the alignment devices can be used for insertion of femoral stem in total hip replacement, where one sensor is mounted on the femur and the other is mounted on the handle which is used to insert the femoral stem. It can also be used in elbow surgery to determine the relationship between the humerus and forearm, and to objectively document the range of movement. It can also be used in trauma surgery, where documentation of range of movement is required, with one sensor mounted on either side of the joint to be tested.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprise" (and any form of comprise, such as "comprises" and "comprising"), "have" (and any form of have, such as "has", and "having"), "include" (and any form of include, such as "includes" and "including"), and "contain" (and any form of contain, such as "contains" and "containing") are open-ended linking verbs. As a result, a method or device that "comprises," "has," "includes," or "contains" one or more steps or elements possesses those one or more steps or elements, but is not limited to possessing only those one or more steps or elements. Likewise, a step of a method or an element of a device that "comprises," "has," "includes," or "contains" one or more features possesses those one or more features, but is not limited to possessing only those one or more features. Furthermore, a device or structure that is configured in a certain way is configured in at least that way, but may also be configured in ways that are not listed.

The invention has been described with reference to the preferred embodiments. It will be understood that the architectural and operational embodiments described herein are exemplary of a plurality of possible arrangements to provide the same general features, characteristics, and general system operation. Attention is directed to the claims hereinafter appearing which define the scope of the invention.

## Claims

1. An alignment device (20; 30) for aligning femoral and tibial bones (1, 2) at a knee joint (3), the device comprising:
a femoral portion (22; 32) to be coupled to the femoral bone (1), and comprising either an intramedullary rod sensor (4) or a flat device comprising an extramedullary sensor (10); and
a tibial portion (24) to be mounted on the tibial bone(2), and comprising a sensor rod (5) mounted on two clamps (8) and including a sensor on the tibial portion (24) to enable determination of femoral and tibial axis in relation to each other.

2. The alignment device of claim 1, wherein the femoral portion (22; 32) and the tibial portion (24) are connectable through wires.

3. The alignment device of claim 1, wherein the femoral portion (22; 32) and the tibial portion (24) are connectable wirelessly.

4. The alignment device of claim 1, wherein a long axis of the femoral portion (22; 32) is parallel to the plane of the long axis of the femoral shaft.

5. The alignment device of claim 1, wherein a long axis of the tibial portion (24) is parallel to the plane of the long axis of the tibial shaft.

6. The alignment device of claim 1, wherein the femoral (22; 32) and tibial (24) portions are made from a material selected from a sterilisable material and a disposable material.

7. The alignment device of claim 1, wherein the sensor on the tibial portion (24) comprises:
at least one of a three-axis accelerometer and a three-axis gyroscope.

8. The alignment device of claim 1, wherein the alignment device (20; 30) further comprises:
a display (6) and wherein the display (6) is wirelessly coupled to the sensor.

9. The alignment device of claim 1, wherein at least one of the sensors (4, 10) is configured to monitor orientation in a three-dimensional coordinate reference system and to generate orientation data corresponding to the orientation of sensor (4) or sensor 10), and sensor rod (5) is configured to monitor orientation in the three dimensional coordinate reference system and to generate orientation data corresponding to the orientation of the sensor rod (5), whereby the relative alignment of a femoral and tibial axis is recordable.

10. The alignment device of claim 1, wherein at least one sensor (4; 10) comprises:
at least one of a three-axis accelerometer and a three-axis gyroscope.

11. The alignment device of claim 1, wherein the at least one sensor (4; 10) comprises:
a housing for holding the sensor.

12. The alignment device of claim 1, wherein at least one additional sensor (12) is provided as a freehand sensor (12).

13. The alignment device of claim 12, wherein at least one additional sensor (12) comprises:
at least one of a three-axis accelerometer and a three-axis gyroscope.

14. The alignment device of claim 9, wherein the at least one sensor (10) is a femoral extramedullary flat sensor (10) which is provided with a clamp (11) for coupling the flat sensor (10) to the femoral bone.

15. The alignment device of claim 14, comprising both an intramedullary rod sensor (4) and a femoral extramedullary sensor (10) positioned such that the extramedullary sensor (10) is alignable with the intramedullary sensor (4) and each of the intramedullary rod sensor (4) and a femoral extramedullary sensor (10) is detectable individually, or in combination, by the tibial rod sensor (5).

16. An alignment device (20; 30) for aligning first and second bones (1, 2) at a joint (3), the device comprising:
a first portion (22; 32) to be coupled to the first bone (1), and comprising either an intramedullary rod sensor (4) or a flat device comprising an extramedullary sensor (10); and
a second portion (24) to be mounted on the second bone (2), and comprising a sensor rod (5) mounted on two clamps (8) and including a sensor on the second portion (24) to enable determination of first bone and second bone axis in relation to each other.

## Patentansprüche

1. Eine Ausrichtungsvorrichtung (20; 30) zum Ausrichten eines femoralen und eines tibialen Knochens (1, 2) an einem Kniegelenk (3), wobei die Vorrichtung Folgendes beinhaltet:
einen femoralen Abschnitt (22; 32), der mit dem femoralen Knochen (1) gekoppelt werden soll und entweder einen intramedullären Stabsensor (4) oder eine flache Vorrichtung, die einen extramedullären Sensor (10) beinhaltet, beinhaltet; und
einen tibialen Abschnitt (24), der auf dem tibialen Knochen (2) befestigt werden soll und einen Sensorstab (5) beinhaltet, der an zwei Halterungen (8) befestigt ist und einen Sensor auf dem tibialen Abschnitt (24) umfasst, um die Bestimmung der femoralen und tibialen Achse in Bezug aufeinander zu ermöglichen.

2. Ausrichtungsvorrichtung gemäß Anspruch 1, wobei der femorale Abschnitt (22; 32) und der tibiale Abschnitt (24) durch Drähte verbunden werden können.

3. Ausrichtungsvorrichtung gemäß Anspruch 1, wobei der femorale Abschnitt (22; 32) und der tibiale Abschnitt (24) drahtlos verbunden werden können.

4. Ausrichtungsvorrichtung gemäß Anspruch 1, wobei eine Längsachse des femoralen Abschnitts (22; 32) parallel zu der Ebene der Längsachse des femoralen Schafts ist.

5. Ausrichtungsvorrichtung gemäß Anspruch 1, wobei eine Längsachse des tibialen Abschnitts (24) parallel zu der Ebene der Längsachse des tibialen Schafts ist.

6. Ausrichtungsvorrichtung gemäß Anspruch 1, wobei der femorale (22; 32) und der tibiale (24) Abschnitt aus einem Material gefertigt sind, das aus einem sterilisierbaren Material und einem wegwerfbaren Material ausgewählt ist.

7. Ausrichtungsvorrichtung gemäß Anspruch 1, wobei der Sensor auf dem tibialen Abschnitt (24) Folgendes beinhaltet:
mindestens eines von einem dreiachsigen Beschleunigungsmesser und einem dreiachsigen Gyroskop.

8. Ausrichtungsvorrichtung gemäß Anspruch 1, wobei die Ausrichtungsvorrichtung (20; 30) ferner Folgendes beinhaltet:
eine Anzeige (6), und wobei die Anzeige (6) drahtlos mit dem Sensor gekoppelt ist.

9. Ausrichtungsvorrichtung gemäß Anspruch 1, wobei mindestens einer der Sensoren (4, 10) konfiguriert ist, um die Orientierung in einem dreidimensionalen Koordinatenreferenzsystem zu überwachen und Orientierungsdaten, die der Orientierung von Sensor (4) oder Sensor (10) entsprechen, zu erzeugen, und der Sensorstab (5) konfiguriert ist, um die Orientierung in dem dreidimensionalen Koordinatenreferenzsystem zu überwachen und Orientierungsdaten, die der Orientierung des Sensorstabs (5) entsprechen, zu erzeugen, wodurch die relative Ausrichtung einer femoralen und einer tibialen Achse aufgezeichnet werden kann.

10. Ausrichtungsvorrichtung gemäß Anspruch 1, wobei mindestens ein Sensor (4; 10) Folgendes beinhaltet:
mindestens eines von einem dreiachsigen Beschleunigungsmesser und einem dreiachsigen Gyroskop.

11. Ausrichtungsvorrichtung gemäß Anspruch 1, wobei der mindestens eine Sensor (4; 10) Folgendes beinhaltet:
ein Gehäuse zum Halten des Sensors.

12. Ausrichtungsvorrichtung gemäß Anspruch 1, wobei mindestens ein zusätzlicher Sensor (12) als ein Freihandsensor (12) bereitgestellt ist.

13. Ausrichtungsvorrichtung gemäß Anspruch 12, wobei mindestens ein zusätzlicher Sensor (12) Folgendes beinhaltet:
mindestens eines von einem dreiachsigen Beschleunigungsmesser und einem dreiachsigen Gyroskop.

14. Ausrichtungsvorrichtung gemäß Anspruch 9, wobei der mindestens eine Sensor (10) ein femoraler extramedullärer flacher Sensor (10) ist, der mit einer Halterung (11) zum Koppeln des flachen Sensors (10) mit dem femoralen Knochen versehen ist.

15. Ausrichtungsvorrichtung gemäß Anspruch 14, die sowohl einen intramedullären Stabsensor (4) als auch einen femoralen extramedullären Sensor (10) beinhaltet, die so positioniert sind, dass der extramedulläre Sensor (10) nach dem intramedullären Sensor (4) ausgerichtet werden kann und jeder von dem intramedullären Stabsensor (4) und einem femoralen extramedullären Sensor (10) individuell oder in Kombination durch den tibialen Stabsensor (5) detektiert werden kann.

16. Eine Ausrichtungsvorrichtung (20; 30) zum Ausrichten erster und zweiter Knochen (1, 2) an einem Gelenk (3), wobei die Vorrichtung Folgendes beinhaltet:
einen ersten Abschnitt (22; 32), der mit dem ersten Knochen (1) gekoppelt werden soll und entweder einen intramedullären Stabsensor (4) oder eine flache Vorrichtung, die einen extramedullären Sensor (10) beinhaltet, beinhaltet; und
einen zweiten Abschnitt (24), der auf dem zweiten Knochen (2) befestigt werden soll und einen Sensorstab (5) beinhaltet, der an zwei Halterungen (8) befestigt ist und einen Sensor auf dem zweiten Abschnitt (24) umfasst, um die Bestimmung der ersten Knochenachse und der zweiten Knochenachse in Bezug aufeinander zu ermöglichen.

## Revendications

1. Un dispositif d'alignement (20 ; 30) pour aligner des os fémoral et tibial (1, 2) au niveau d'une articulation de genou (3), le dispositif comprenant :
une portion fémorale (22 ; 32) devant être couplée à l'os fémoral (1), et comprenant soit un capteur à tige intramédullaire (4), soit un dispositif plat comprenant un capteur extramédullaire (10) ; et
une portion tibiale (24) devant être montée sur l'os tibial (2), et comprenant une tige de capteur (5) montée sur deux pinces (8) et incluant un capteur sur la portion tibiale (24) afin de permettre une détermination d'un axe fémoral et tibial l'un par rapport à l'autre.

2. Le dispositif d'alignement de la revendication 1, dans lequel la portion fémorale (22 ; 32) et la portion tibiale (24) peuvent être connectées par l'intermédiaire de fils.

3. Le dispositif d'alignement de la revendication 1, dans lequel la portion fémorale (22 ; 32) et la portion tibiale (24) peuvent être connectées sans fil.

4. Le dispositif d'alignement de la revendication 1, dans lequel un axe long de la portion fémorale (22 ; 32) est parallèle au plan de l'axe long de la diaphyse fémorale.

5. Le dispositif d'alignement de la revendication 1, dans lequel un axe long de la portion tibiale (24) est parallèle au plan de l'axe long de la diaphyse tibiale.

6. Le dispositif d'alignement de la revendication 1, dans lequel les portions fémorale (22 ; 32) et tibiale (24) sont fabriquées à partir d'un matériau sélectionné parmi un matériau stérilisable et un matériau jetable.

7. Le dispositif d'alignement de la revendication 1, dans lequel le capteur sur la portion tibiale (24) comprend :
au moins un élément parmi un accéléromètre à trois axes et un gyroscope à trois axes.

8. Le dispositif d'alignement de la revendication 1, le dispositif d'alignement (20 ; 30) comprenant en sus :
un organe d'affichage (6) et dans lequel l'organe d'affichage (6) est couplé sans fil au capteur.

9. Le dispositif d'alignement de la revendication 1, dans lequel au moins un des capteurs (4, 10) est configuré pour surveiller une orientation dans un système de référence de coordonnées tridimensionnel et pour générer des données d'orientation correspondant à l'orientation du capteur (4) ou du capteur (10), et la tige de capteur (5) est configurée pour surveiller une orientation dans le système de référence de coordonnées tridimensionnel et pour générer des données d'orientation correspondant à l'orientation de la tige de capteur (5), moyennant quoi l'alignement relatif d'un axe fémoral et tibial est enregistrable.

10. Le dispositif d'alignement de la revendication 1, dans lequel au moins un capteur (4 ; 10) comprend :
au moins un élément parmi un accéléromètre à trois axes et un gyroscope à trois axes.

11. Le dispositif d'alignement de la revendication 1, dans lequel l'au moins un capteur (4 ; 10) comprend :
un logement pour maintenir le capteur.

12. Le dispositif d'alignement de la revendication 1, dans lequel au moins un capteur supplémentaire (12) est fourni en tant que capteur main-libre (12).

13. Le dispositif d'alignement de la revendication 12, dans lequel au moins un capteur supplémentaire (12) comprend :
au moins un élément parmi un accéléromètre à trois axes et un gyroscope à trois axes.

14. Le dispositif d'alignement de la revendication 9, dans lequel l'au moins un capteur (10) est un capteur plat extramédullaire fémoral (10) qui est muni d'une pince (11) pour coupler le capteur plat (10) à l'os fémoral.

15. Le dispositif d'alignement de la revendication 14, comprenant à la fois un capteur à tige intramédullaire (4) et un capteur extramédullaire fémoral (10) positionnés de telle sorte que le capteur extramédullaire (10) est alignable avec le capteur intramédullaire (4) et que chaque capteur parmi le capteur à tige intramédullaire (4) et un capteur extramédullaire fémoral (10) est détectable individuellement, ou en combinaison, par le capteur à tige tibial (5).

16. Un dispositif d'alignement (20 ; 30) pour aligner des premier et deuxième os (1, 2) au niveau d'une articulation (3), le dispositif comprenant :
une première portion (22 ; 32) devant être couplée au premier os (1), et comprenant soit un capteur à tige intramédullaire (4), soit un dispositif plat comprenant un capteur extramédullaire (10) ; et
une deuxième portion (24) devant être montée sur le deuxième os (2), et comprenant une tige de capteur (5) montée sur deux pinces (8) et incluant un capteur sur la deuxième portion (24) afin de permettre une détermination d'un axe de premier os et de deuxième os l'un par rapport à l'autre.
